# EUROPEAN PATENT APPLICATION

(11) **EP 2 258 865 A1**
(43) Date of publication of application: **08.12.2010**
(21) Application number: 09162122.7
(22) Date of filing: 05.06.2009
(51) Int. Cl.: C12Q 1/68, G01N 33/574, A61K 31/00, A61P 35/00

(54) **Lysine-specific demethylase 1 (LSD1) is a biomarker for breast cancer**

(71) Applicant: Universitätsklinikum Freiburg, 79106 Freiburg (DE)
(72) Inventor: Schüle, Roland, 79367 Weisweil (DE); Metzger, Eric, 68600 Neuf-Brisach (FR); Kirfel, Jutta, 53125 Bonn (DE); Büttner, Reinhard, 53343 Villiprot (DE)
(74) Representative: Bühler, Dirk

(57) **Abstract**

The present invention relates to novel biomarker for breast cancer, namely LSD1, and its application in *inter alia* the diagnosis of breast cancer. Furthermore, the present invention discloses a method of determining the LSD1 protein amount and the effect of LSD1 inhibitors on cancer cells selected from breast cancer, lung carcinoma and sarcoma.

SEQUENCE LISTING

<110> Universitätsklinikum Freiburg

<120> Lysine-specific demethylase 1 (LSD1) is a biomarker for breast
      cancer

<130> U 7131 / DB

<160> 5

<170> PatentIn version 3.3

<210> 1
<211> 20
<212> DNA
<213> Artificial

<220>
<223> primer sequence used for RT-PCR, forward primer to detect CCNA2

<400> 1
cctgctcagt ttcctttggt                                                   20


<210> 2
<211> 20
<212> DNA
<213> Artificial

<220>
<223> primer sequence used for RT-PCR, reverse primer to detect CCNA2

<400> 2
atcccgcgac tattgaaatg                                                   20


<210> 3
<211> 20
<212> DNA
<213> artificial

<220>
<223> primer sequence used for RT-PCR, forward primer to detect ERBB2

<400> 3
ggcttgggat ggagtaggat                                                   20


<210> 4
<211> 20
<212> DNA
<213> artificial

<220>
<223> primer sequence used for RT-PCR, reverse primer to detect ERBB2

<400> 4
tccctaggct gccactctta                                                   20


<210> 5
<211> 852
<212> PRT
<213> Homo sapiens

<400> 5
   Met Leu Ser Gly Lys Lys Ala Ala Ala Ala Ala Ala Ala Ala Ala Ala
   1               5                   10                  15
   Ala Ala Thr Gly Thr Glu Ala Gly Pro Gly Thr Ala Gly Gly Ser Glu
               20                  25                  30
   Asn Gly Ser Glu Val Ala Ala Gln Pro Ala Gly Leu Ser Gly Pro Ala
           35                  40                  45
   Glu Val Gly Pro Gly Ala Val Gly Glu Arg Thr Pro Arg Lys Lys Glu
       50                  55                  60
   Pro Pro Arg Ala Ser Pro Pro Gly Gly Leu Ala Glu Pro Pro Gly Ser
   65                  70                  75                  80
   Ala Gly Pro Gln Ala Gly Pro Thr Val Val Pro Gly Ser Ala Thr Pro
                   85                  90                  95
   Met Glu Thr Gly Ile Ala Glu Thr Pro Glu Gly Arg Arg Thr Ser Arg
               100                 105                 110
   Arg Lys Arg Ala Lys Val Glu Tyr Arg Glu Met Asp Glu Ser Leu Ala
           115                 120                 125
   Asn Leu Ser Glu Asp Glu Tyr Tyr Ser Glu Glu Glu Arg Asn Ala Lys
       130                 135                 140
   Ala Glu Lys Glu Lys Lys Leu Pro Pro Pro Pro Pro Gln Ala Pro Pro
   145                 150                 155                 160
   Glu Glu Glu Asn Glu Ser Glu Pro Glu Glu Pro Ser Gly Val Glu Gly
                   165                 170                 175
   Ala Ala Phe Gln Ser Arg Leu Pro His Asp Arg Met Thr Ser Gln Glu
               180                 185                 190
   Ala Ala Cys Phe Pro Asp Ile Ile Ser Gly Pro Gln Gln Thr Gln Lys
           195                 200                 205
   Val Phe Leu Phe Ile Arg Asn Arg Thr Leu Gln Leu Trp Leu Asp Asn
       210                 215                 220
   Pro Lys Ile Gln Leu Thr Phe Glu Ala Thr Leu Gln Gln Leu Glu Ala
   225                 230                 235                 240
   Pro Tyr Asn Ser Asp Thr Val Leu Val His Arg Val His Ser Tyr Leu
                   245                 250                 255
   Glu Arg His Gly Leu Ile Asn Phe Gly Ile Tyr Lys Arg Ile Lys Pro
               260                 265                 270
   Leu Pro Thr Lys Lys Thr Gly Lys Val Ile Ile Ile Gly Ser Gly Val
           275                 280                 285
   Ser Gly Leu Ala Ala Ala Arg Gln Leu Gln Ser Phe Gly Met Asp Val
       290                 295                 300
   Thr Leu Leu Glu Ala Arg Asp Arg Val Gly Gly Arg Val Ala Thr Phe
   305                 310                 315                 320
   Arg Lys Gly Asn Tyr Val Ala Asp Leu Gly Ala Met Val Val Thr Gly
                   325                 330                 335
   Leu Gly Gly Asn Pro Met Ala Val Val Ser Lys Gln Val Asn Met Glu
               340                 345                 350
   Leu Ala Lys Ile Lys Gln Lys Cys Pro Leu Tyr Glu Ala Asn Gly Gln
           355                 360                 365
   Ala Val Pro Lys Glu Lys Asp Glu Met Val Glu Gln Glu Phe Asn Arg
       370                 375                 380
   Leu Leu Glu Ala Thr Ser Tyr Leu Ser His Gln Leu Asp Phe Asn Val
   385                 390                 395                 400
   Leu Asn Asn Lys Pro Val Ser Leu Gly Gln Ala Leu Glu Val Val Ile
                   405                 410                 415
   Gln Leu Gln Glu Lys His Val Lys Asp Glu Gln Ile Glu His Trp Lys
               420                 425                 430
   Lys Ile Val Lys Thr Gln Glu Glu Leu Lys Glu Leu Leu Asn Lys Met
           435                 440                 445
   Val Asn Leu Lys Glu Lys Ile Lys Glu Leu His Gln Gln Tyr Lys Glu
       450                 455                 460
   Ala Ser Glu Val Lys Pro Pro Arg Asp Ile Thr Ala Glu Phe Leu Val
   465                 470                 475                 480
   Lys Ser Lys His Arg Asp Leu Thr Ala Leu Cys Lys Glu Tyr Asp Glu
                   485                 490                 495
   Leu Ala Glu Thr Gln Gly Lys Leu Glu Glu Lys Leu Gln Glu Leu Glu
               500                 505                 510
   Ala Asn Pro Pro Ser Asp Val Tyr Leu Ser Ser Arg Asp Arg Gln Ile
           515                 520                 525
   Leu Asp Trp His Phe Ala Asn Leu Glu Phe Ala Asn Ala Thr Pro Leu
       530                 535                 540
   Ser Thr Leu Ser Leu Lys His Trp Asp Gln Asp Asp Asp Phe Glu Phe
   545 550 555 560
   Thr Gly Ser His Leu Thr Val Arg Asn Gly Tyr Ser Cys Val Pro Val
   565 570 575
   Ala Leu Ala Glu Gly Leu Asp Ile Lys Leu Asn Thr Ala Val Arg Gln
   580 585 590
   Val Arg Tyr Thr Ala Ser Gly Cys Glu Val Ile Ala Val Asn Thr Arg
   595 600 605
   Ser Thr Ser Gln Thr Phe Ile Tyr Lys Cys Asp Ala Val Leu Cys Thr
   610 615 620
   Leu Pro Leu Gly Val Leu Lys Gln Gln Pro Pro Ala Val Gln Phe Val
   625 630 635 640
   Pro Pro Leu Pro Glu Trp Lys Thr Ser Ala Val Gln Arg Met Gly Phe
   645 650 655
   Gly Asn Leu Asn Lys Val Val Leu Cys Phe Asp Arg Val Phe Trp Asp
   660 665 670
   Pro Ser Val Asn Leu Phe Gly His Val Gly Ser Thr Thr Ala Ser Arg
   675 680 685
   Gly Glu Leu Phe Leu Phe Trp Asn Leu Tyr Lys Ala Pro Ile Leu Leu
   690 695 700
   Ala Leu Val Ala Gly Glu Ala Ala Gly Ile Met Glu Asn Ile Ser Asp
   705 710 715 720
   Asp Val Ile Val Gly Arg Cys Leu Ala Ile Leu Lys Gly Ile Phe Gly
   725 730 735
   Ser Ser Ala Val Pro Gln Pro Lys Glu Thr Val Val Ser Arg Trp Arg
   740 745 750
   Ala Asp Pro Trp Ala Arg Gly Ser Tyr Ser Tyr Val Ala Ala Gly Ser
   755 760 765
   Ser Gly Asn Asp Tyr Asp Leu Met Ala Gln Pro Ile Thr Pro Gly Pro
   770 775 780
   Ser Ile Pro Gly Ala Pro Gln Pro Ile Pro Arg Leu Phe Phe Ala Gly
   785 790 795 800
   Glu His Thr Ile Arg Asn Tyr Pro Ala Thr Val His Gly Ala Leu Leu
   805 810 815
   Ser Gly Leu Arg Glu Ala Gly Arg Ile Ala Asp Gln Phe Leu Gly Ala
   820 825 830
   Met Tyr Thr Leu Pro Arg Gln Ala Thr Pro Gly Val Pro Ala Gln Gln
   835 840 845
   Ser Pro Ser Met
   850

## Description

### FIELD OF THE INVENTION

The present invention relates to a method of diagnosing breast cancer and/or substantiating the diagnosis of breast cancer and to a method of determining the course of breast cancer in a subject, wherein said methods are *inter alia* based on the determination of the expression level of LSD1. The present invention also relates to a method of determining the amount of the LSD1 protein in a sample as well as to the use of a pharmaceutical composition comprising an LSD1-inhibitor for the treatment of a cancer selected from breast cancer, lung carcinoma and sarcoma.

### BACKGROUND OF THE INVENTION

Cancer may affect people at all ages and causes about 13% of all deaths. According to the American Cancer Society, 7.6 million people died from cancer in the world during 2007.

Almost all cancers are caused by abnormalities in the genetic material of the transformed cells. Typically, said abnormalities affect two main classes of genes: cancer-promoting oncogenes are typically activated in cancer cells resulting in new properties of said cells, such as hyperactive growth and division; tumor suppressor genes, on the other hand, are often found to be inactivated in cancer cells, resulting in the loss of strictly regulated processes in cancer cells, such as accurate DNA replication or control over the cell cycle.

Among women in the industrialized countries of the Western societies, breast cancer represents the most commonly diagnosed tumor. Together with lung cancer, breast cancer is the most important cause of cancer-associated morbidity and mortality.

Two main objectives in cancer research are thus directed to the following areas: the diagnosis and/or the characterization of cancer and the treatment of cancer. Often, said objectives seem to be connected via the molecular cause of cancer.

For the diagnosis and/or the characterization of cancer, research is *inter alia* focused on so-called "biomarkers", such as proteins or specific nucleic acids sequences, which seem to correlate with cancer or with a specific type of cancer, respectively. Examples for biomarkers in breast cancer are the estrogen receptor (ER) and the progesterone receptor (PR)-status and HER2-expression and gene amplification.

Such biomarkers are often used for the decision, which therapeutic steps should be initiated in the individual patient in order to treat the identified type of cancer. Examples for such treatments of breast cancer are hormone therapies in ER-positive breast cancers or, in case of HER2-overexpression, therapies directed to the inhibition of HER2 using antibodies and/or specific inhibitors.

Thus, it is evident that a correlation between a biomarker and a treatment regimen can exist in cases, wherein a molecular function of the biomarker seems to correlate with an unregulated process in a cancer cell.

Over the past years, it became evident that not only members of the classical signaling pathways implicated in cancers may serve as biomarkers and targets, but that also so-called "epigenetic marks" may be used in order to predict and/or diagnose cancer. Said epigenetic marks are mainly found on histones of the DNA. Particularly in DNA regions implicated in gene regulation, there seems to be a highly diverse, interconnected and remarkably controlled set of stable and transient epigenetic marks comprising *inter alia* histone- phosphorylation, -methylation, - acetylation, -ubiquitylation and -SUMOylation [Jenuwein, T. et al. (2001) Translating the histone code. Science, 293, 1074-1080].

Recently, enzymes responsible for the above-mentioned epigenetic marks have been identified; in this respect, lysine specific demethylase 1 (LSD1) was identified [e.g. Metzger, E. et al. (2005) LSD1 demethylates repressive histone marks to promote androgen-receptor-dependent transcription. Nature, 437, 436-439] and its role characterized in neuroblastoma [Schulte, J.H. et al. (2009) Lysine-specific demethylase 1 is strongly expressed in poorly differentiated neuroblastoma: implications for therapy. Cancer Res., 69, 2065-2071].

However, due to the complexity of the "disease cancer", wherein results among different types of cancer vary to a large extent, there is an ongoing need for novel biomarkers indicative of and indicative for the course of a specific cancer type such as breast cancer; furthermore, there is an ongoing need for new therapeutic approaches for cancer in general and for specific cancer types.

### OBJECTS AND SUMMARY OF THE INVENTION

It is one object of the present invention to provide a method of diagnosing breast cancer and/or substantiating the diagnosis of breast cancer in a subject.

It is another object of the present invention to provide a method of determining the course of breast cancer in a subject.

Furthermore, it is an object of the present invention to provide a method of determining the amount and/or concentration of the LSD1 protein in a sample.

It is then among the further objects of the present invention to provide the use of a pharmaceutical composition comprising an LSD1-inhibitor for the treatment of a cancer selected from the group of cancers comprising breast cancer, lung carcinoma and sarcoma.

These and other objects as they will become apparent from the ensuing description are attained by the subject-matter of the independent claims. The dependent claims relate to some of the preferred embodiments of the present invention.

The inventors have surprisingly found that the expression level of lysine specific demethylase 1 (LSD1) can be used to diagnose breast cancer and/or substantiate the diagnosis of breast cancer as well as to determine the course of breast cancer in a subject. Concerning the activity of LSD1, the inventors have furthermore surprisingly found that the inhibition of LSD1 results in growth arrest of tumor cells selected from breast cancer cells, lung carcinoma cells and sarcoma cells, and may thus be used as therapeutic approach of treating said cancers.

As mentioned above, the present invention is in one aspect concerned with a method of diagnosing breast cancer and/or substantiating the diagnosis of breast cancer in a subject. Said method comprises at least the steps of:
a) Providing a sample of breast tissue of said subject outside the human or animal body;
b) Determining the expression level n1 of lysine specific demethylase 1 (LSD1) in said sample;
c) Comparing the expression level n1 obtained in step b) to a reference expression level n2 of LSD1 in a sample of breast tissue of a healthy subj ect;
d) Diagnosing breast cancer and/or substantiating the diagnosis of breast cancer based on said comparison, wherein n1 > n2 is indicative of breast cancer.

In a preferred embodiment of this object of the invention, said sample of breast tissue provided in step a) is tissue suspicious of being breast cancer tissue.

In another preferred embodiment of this object, said sample of breast tissue is derived from a surgery or a biopsy of said subject.

In an also preferred embodiment of this object of the invention, n1 > n2 is indicative of breast cancer characterized by the absence of estrogen receptor (ER negative). Thus, ER negative breast cancer may be diagnosed or the diagnosis of ER negative breast cancer may be substantiated by a method of this first object of the present invention.

In a second aspect, the present invention is concerned with a method of determining the course of breast cancer in a subject. Said method comprises at least the steps of:
a) Providing a sample of breast tissue of said subject outside the human or animal body;
b) Determining the expression level n1 of lysine specific demethylase 1 (LSD1) in said sample;
c) Comparing the expression level n1 obtained in step b) to a reference expression level n2 of LSD1 in a sample of breast tissue of a healthy subj ect;
d) Determining the course of breast cancer based on said comparison, wherein n1 > n2 is indicative of an aggressive biology of said breast cancer.

In a preferred embodiment of this aspect of the invention, said sample of breast tissue provided in step a) is breast cancer tissue.

In an even more preferred embodiment, said breast cancer tissue is selected from ductal or lobular breast cancer and breast cancer classified according to the St.-Gallen criteria as being of high or intermediate or low risk.

In another preferred embodiment of this object of the invention, said sample of breast cancer tissue is derived from a surgery or a biopsy of said subject.

In an also preferred embodiment of this aspect of the invention, the aggressive biology of said breast cancer correlates with n1, i.e. the higher n1, the more aggressive the breast cancer.

In an also preferred embodiment of both aspects of the invention mentioned above, the reference expression level n2 of LSD1 in a sample of breast tissue of a healthy subject mentioned in step c) represents the result of a large control group of healthy subjects, preferably of about 10000, about 1000, about 500, about 100 or about 50 healthy subjects.

Furthermore, in another preferred embodiment of both above-mentioned aspects, said subject is a female subject, preferably a female human being.

In both aspects mentioned above, the expression level of LSD1 in a sample is determined. In the following, preferred embodiments relating to said determination will be outlined. In this regard, it needs to be understood that an expression level of LSD1 may be expression in different ways, e.g. by way of stating the amount and/or the concentration of the LSD1 species analyzed comprising e.g. the LSD1 gene, the LSD1 mRNA or the LSD1 protein. Furthermore, it needs to be understood that the expression level is always normalized to a second parameter, e.g. the overall amount or concentration or weight of the sample or the species analyzed. Finally, it needs to be understood that this refers to the sample mentioned in step a), i.e. the sample to be analyzed, as well as to the sample mentioned in step c), i.e. the reference sample. Obviously, said two samples and thus the two expression levels n1 and n2 can be compared only if they are both expressed in an identical way regarding their unit and/or their normalization.

Thus, in a preferred embodiment of the present invention, the expression level of LSD1 is determined by analyzing the LSD1 gene and/or by analyzing the mRNA transcribed from the LSD1 gene and/or by analyzing the LSD1 protein.

In a preferred embodiment, the LSD1 gene is analyzed for its gene copy number using a method selected from the group of methods comprising quantitative hybridization techniques using oligonucleotide probes directed to the LSD1 gene, quantitative sequencing analysis and combinations thereof. Thus, the expression level may be referred to as the number of LSD1 genes present in the genomic DNA of a sample.

Preferably, the LSD1 gene analyzed comprises DNA regions coding for LSD1 and DNA regions regulating the LSD1 expression. Thus, in another preferred embodiment, the DNA regions regulating the LSD1 expression may be analyzed for at least one mutation and/or at least one epigenetic mark resulting in an aberrant LSD1 expression. Said analysis may be carried out using methods selected from the group of methods comprising sequencing techniques, GenoSnip methods in order to identify point mutations, Western-blot analysis in order to analyze the epigenetic marks, Chromatin-Immunoprecipitations, Immunoprecipitations, hybridization techniques and combinations thereof. Thus, the expression level may also be referred to as aberrant LSD1 expression comprising either upregulation or downregulation of the LSD1 gene in the genomic DNA of a sample.

In another preferred embodiment, the mRNA transcribed from the LSD1 gene (LSD1 mRNA) is analyzed for its amount using a method selected from the group of methods comprising quantitative PCR, real-time-PCR, Northern-blot and combinations thereof. Thus, the expression level may be referred to as the amount of LSD1 mRNA present in a sample.

In another preferred embodiment, the LSD1 protein is analyzed for its amount using methods selected from the group of methods comprising an ELISA-assay, Western-blot analysis, mass spectrometry, Immunostaining, Immunoprecipitation, chromatography and combinations thereof. Thus, the expression level may be referred to as the amount of LSD1 protein in a sample.

However, the LSD1 protein may also be analyzed for posttranslational modifications affecting the LSD1 activity. Thus, the determination of the expression level may also comprise the determination of the modification state of LSD1, wherein LSD1 may be modified by phosphorylation, acetylation, ubiquitinylation, SUMOylation, and the like. In a preferred embodiment, the analysis of the at least one posttranslational modification potentially affecting LSD1 activity may be carried out by determining the type and/or amount of the posttranslational modification using e.g. methods such as Western-blots using antibodies directed to modified residues and/or modifications, Immunoprecipitation, Immunostaining, mass spectrometry, and the like. Thus, the expression level may also be referred to as the modification state of the LSD1 protein in a sample.

As mentioned above, the invention is in a third object directed to a method of determining the amount and/or concentration of the LSD1 protein in sample. Said method comprises at least the steps of:
a) Providing a solution of said sample comprising proteins, wherein said proteins are present in a concentration of between about 0.01 mg/ml and about 10 mg/ml, preferably between about 0.05 mg/ml and about 5 mg/ml, more preferably between about 0.1 mg/ml and about 1 mg/ml and most preferably of about 0.4 mg/ml or 0.5 mg/ml in said solution;
b) Coating said proteins onto a surface of a well;
c) Incubating said well with a solution comprising an LSD1-antibody;
d) Incubating said well with a solution comprising a compound specifically binding to said LSD1-antibody, wherein said compound excites a signal upon stimulation;
e) Stimulating said compound;
f) Quantifying the signal excited upon stimulation; and
g) Assigning an amount and/or a concentration to the LSD1 protein in said sample according to the intensity of said signal using a standard curve.

In a preferred embodiment of the third object of the invention, said method comprises additional washing steps after the coating step b) and/or after the incubation step c) and/or after the incubation step d) and/or after the stimulation step e) of the above-mentioned method, wherein said washing steps are preferably carried out several times, preferably about three times.

In an also preferred embodiment of this object of the invention, the standard curve was recorded using recombinant LSD1 and is linear over an amount of LSD1 ranging from about 2.5 ng to about 25 ng corresponding to a concentration range of about 0.025 µg/ml to about 0.25 µg/ml.

In a further preferred embodiment relating to this object, said sample is a tissue sample comprising cells of a subject provided outside the human or animal body or a sample comprising cells cultured *in vitro*, wherein said cells are lysed in order to provide a solution comprising proteins as mentioned in step a).

In an even more preferred embodiment, said sample is a breast tissue sample derived from either breast tissue suspicious of being breast cancer tissue or from breast cancer tissue, wherein said breast cancer tissue is preferably ductal or lobular breast cancer and wherein said sample is provided outside the human or animal body.

In an especially preferred embodiment relating to all three objects mentioned above, the method described in the third object above is used in order to determine the amount of the LSD1 protein in a sample of breast tissue and thus the expression level of LSD1 in said sample when diagnosing breast cancer and/or substantiating the diagnosis of breast cancer or when determining the course of breast cancer in a subject; thus, when a method according to the first and/or second aspect of the invention is carried out.

Furthermore, in a fourth object, the present invention is directed to the use of a pharmaceutical composition comprising an LSD1-inhibitor for the treatment of a cancer selected from the group of cancers consisting of breast cancer, lung carcinoma and sarcoma.

In a preferred embodiment of this aspect of the invention, said cancer is breast cancer, wherein said breast cancer is preferably selected from ductal, lobular, ER positive, ER negative, PR negative, PR positive cancer and breast cancer classified according to the St.-Gallen criteria as being of high or intermediate or low risk.

In an even preferred embodiment of this aspect of the invention, said breast cancer is ER negative and/or PR negative breast cancer.

In an also preferred embodiment of this aspect of the invention, said pharmaceutical composition comprises said LSD1-inhibitor as the only pharmaceutically active agent.

In another preferred embodiment of said fourth object of the invention, said pharmaceutical composition comprises at least one additional pharmaceutically active agent for the treatment of either breast cancer, lung carcinoma or sarcoma. Said at least one additional pharmaceutically active agent is preferably selected from the group comprising Tamoxifen, Fulvestrant (Faslodex®), Trastuzumab (Herceptin®), Lapatinib (Tykerb), Bevacizumab (Avastin), formestane, fadrozole, anastrozole (Arimidex), letrozole (Femara), exemestane (Aromasin), Clodronate, pamidronate, zoledronic acid, ibandronatefor and mixtures thereof for the treatment of breast cancer; from the group comprising erlotinib (Tarceva), bevacizumab (Avastin), topotecan (HYCAMTIN®), paclitaxel (Taxol®), docetaxel (Taxotere®), gemcitabine (Gemzar®), and irinotecan (Camptosar®), cyclophosphamide, doxorubicin (Adriamycin®), vincristine (Oncovin®), paclitaxel, cisplatin (Platinol®) and combinations thereof for the treatment of lung carcinoma; and from the group comprising Imatinib (Glivec) and Trabectedin (Yondelis) and combinations thereof for the treatment of sarcoma.

In an even more preferred embodiment of the fourth aspect of the present invention, said inhibitor is selected from the group of MAO-inhibitors comprising trancylpromine, clorgyline, pargyline, iproclozide, isocarboxazid, mebanazine, nialamide, safrazine, befloxatone, cimoxatone and mixtures thereof.

With respect to the fourth object mentioned above, the present invention may also be formulated as relating to a method of treating cancer selected from the group of cancers consisting of breast cancer, lung carcinoma and sarcoma in a human or animal being comprising administering to a subject, in an amount effective to treat said cancer, an LSD1-inhibitor.

Of course, the above-mentioned preferred embodiments also apply for this formulation of the fourth aspect of the present invention, i.e. that said LSD1-inhibitor may e.g. be the only pharmaceutically active agent administered or may e.g. be administered together with at least one additional pharmaceutically active agent for the treatment of said cancer as mentioned above.

### DESCRIPTION OF THE FIGURES

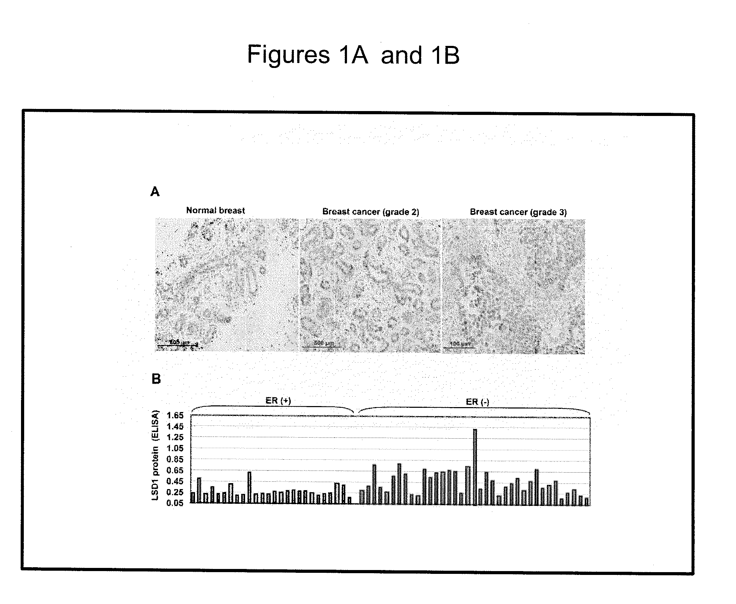

### 1/9: Fig. 1A and B: Expression of LSD1 in breast cancer.

**(A)** Immunohistochemical staining of LSD1 in normal breast tissue and breast cancer (histologic grade 2 and 3). **(B)** LSD1 expression levels in 26 ER-positive and 37 ER-negative breast tumors were analyzed with an ELISA for LSD1. ER (+): ER-positive; ER (-): ER-negative.

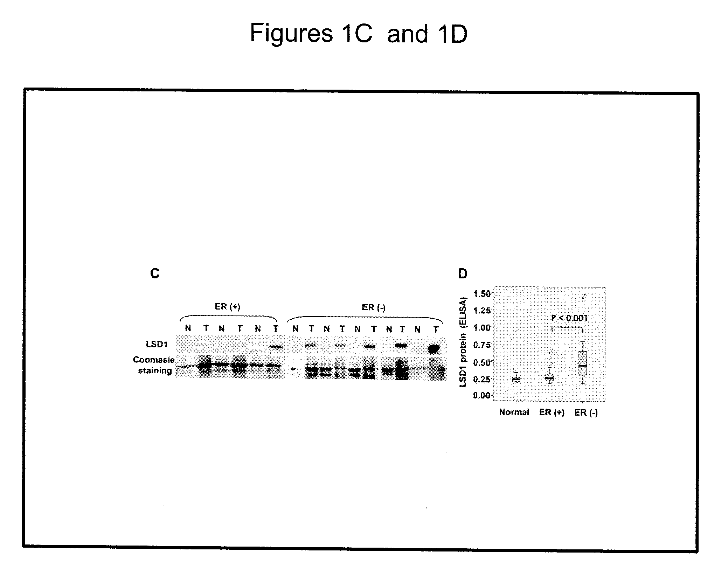

### 2/9: Fig. 1C and D: Expression of LSD1 in breast cancer.

**(C)** LSD1 expression in normal and tumor tissue extracts was determined by western blot. Coomasie staining was used as the loading control. N: normal breast tissue; T: breast tumor tissue. **(D)** Statistical significance test of ELISA was done by two-sided, non-parametrical Mann-Whitney U-test to analyze differences in expression levels among normal, ER-positive and ER-negative groups.

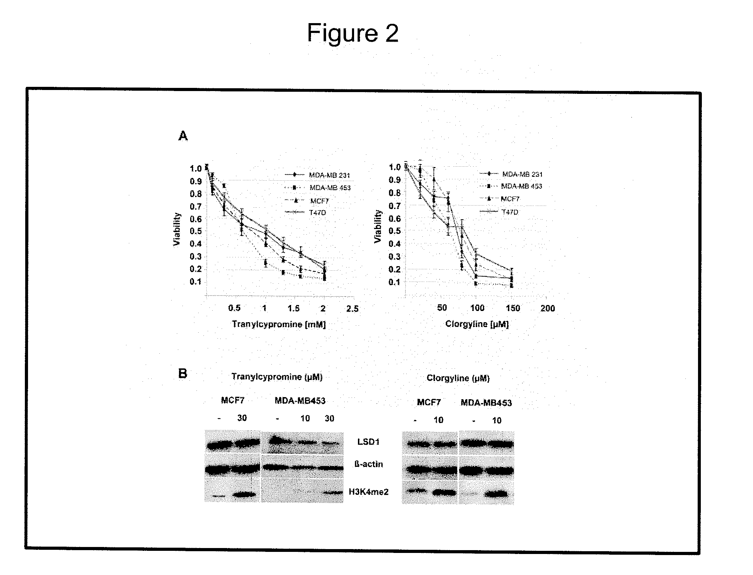

### 3/9: Fig. 2: Reduction in cell growth and increase of global H3K4 methylation upon MAOIs treatment.

**(A)** Four different breast cancer cells were treated with tranylcypromine and clorgyline (MAO-Inhibitors) for 72 h, followed by an MTT assay in order to determine the amount of viable cells. **(B)** Western blot analysis confirmed an accumulation of H3 K4 dimethylation upon treatment with 10 and or 30 µM tranylcypromine and clorgyline for 24 h. LSD1 protein levels were not affected. β-actin served as the loading control.

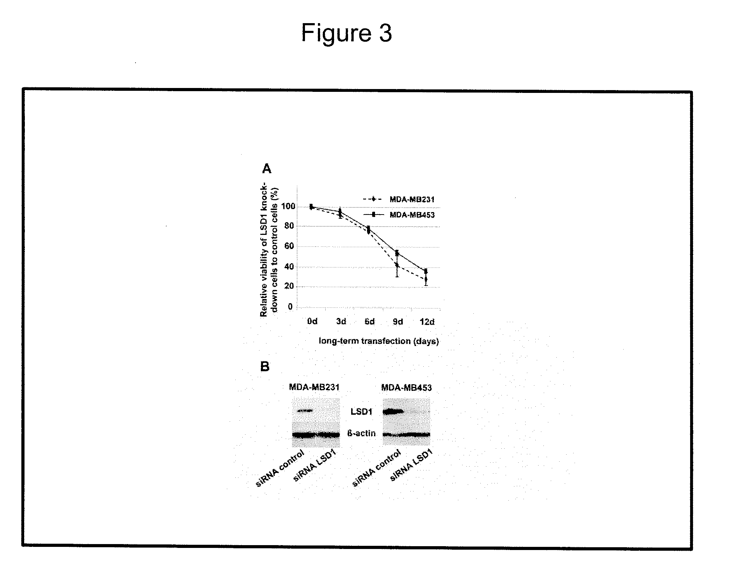

### 4/9: Fig. 3: Decreased cellular growth upon siRNA mediated knock-down of LSD 1.

**(A)** A significant reduction in cell number was observed in an MTT assay upon knock down of LSD1. MDA-MB 231 and MDA-MB 453 cells were treated with siRNA against LSD1 for 12 days. **(B)** Knock-down of LSD1 protein levels was determined 6 days after transfection by western blot. β-actin served as the loading control.

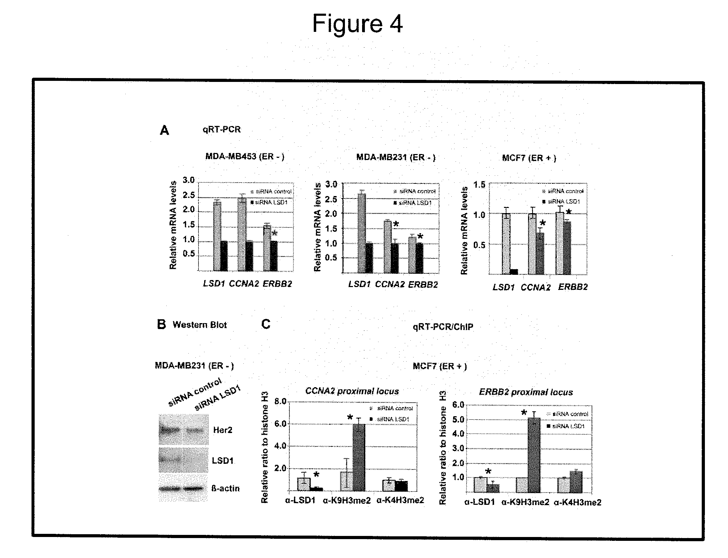

### 5/9: Fig. 4: Down-regulation of CCNA2 and ERBB2 mRNA expression and enrichment of H3K9 di-methylation in the promoter region upon knock-down of LSD1.

**(A)** Quantitative PCR analysis was done in the three different breast cancer cells treated with siRNA directed against LSD1 or with scrambled control siRNA. *CCNA2* and *ERBB2* mRNA expressions were down-regulated 3 days after knock-down of LSD1. 18S rRNA was used as the endogenous reference gene. **(B)** Reduction in Her2/erbB2 protein expression 6 days after knock-down of LSD1 was determined by western blotting. **(C)** Enrichment of H3K9 di-methylation in the proximal promoter region of *CCNA2* or *ERBB2* was observed upon knock down of LSD1 using ChIP assay. The sonificated chromatin of MCF7 cells was immunoprecipitated with α-LSD1, α-K9H3me2 and α-K4H3me2. The precipitated DNA was amplified by PCR using primers flanking the *CCNA2* proximal locus or *ERBB2* proximal locus. **P* < 0.05.

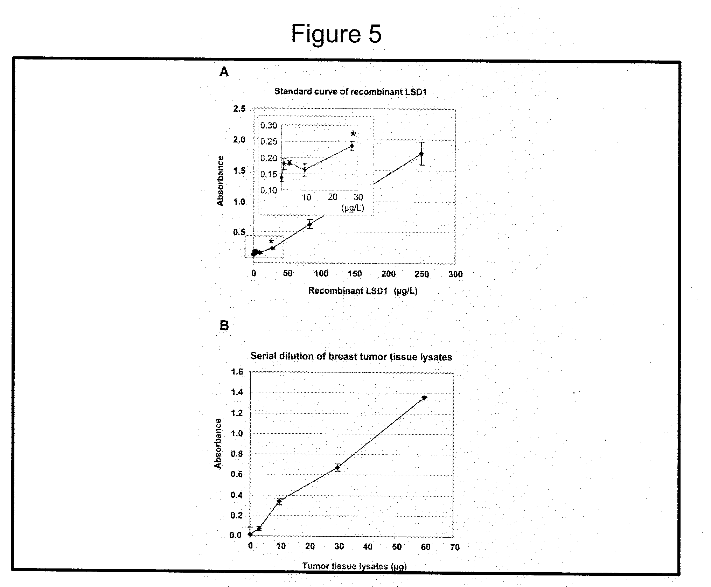

### 6/9: Fig. 5: ELISA-parameters.

**(A)** Dose-response curve of LSD1 ELISA. A series dilution of the purifed His₆-tagged LSD1ΔN ranging from 1 to 250 µg/l (corresponding to an amount of LSD1ΔN used in the series dilution ranging from 0.1 ng to 25 ng) was used as a calibrator in the LSD1 ELISA to generate the dose-response curve. Linear range was 27.8 -250.0 µg/L and the linear correlation coefficient (R²) was 0.99. The detection limit was estimated as the minimum analyte concentration evoking a response significantly different from that of the zero calibrator. The detection limit of the assay was 27.8 µg/l (*P* < 0.01) corresponding to 2.78 ng.
**(B)** Linearity of dilution curves for breast tumor lysates. A series dilution of breast tumor tissue protein lysates was used in the linearity study. In the range of 3 - 60 µg protein lysates (corresponding to a concentration of 0.03 mg/ml to 0.6 mg/ml protein lysates), the dilution curve was close to linear (R²=0.99).

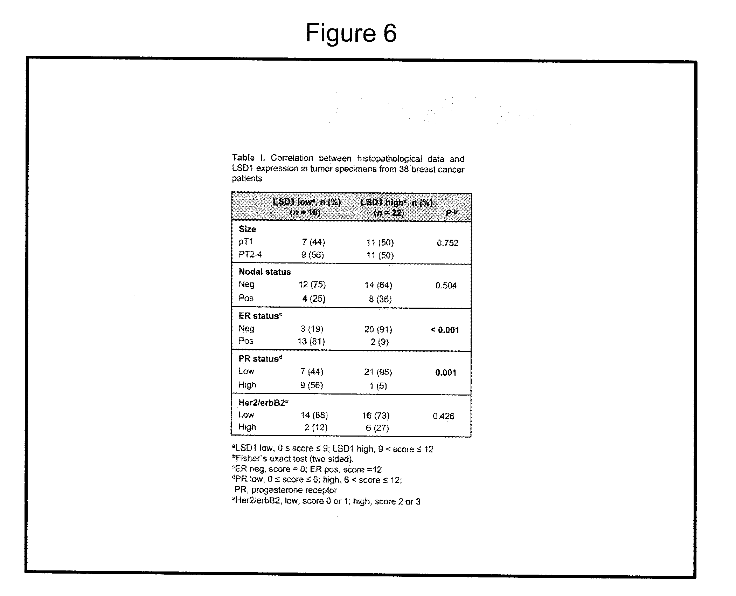

### 7/9: Fig. 6: Correlation between histopathological data and LSD1 expression in tumor specimens from 38 breast cancer patients.

^{a}LSD1 low, 0 ≤ score ≤ 9; LSD1 high, 9 < score ≤ 12;
^{b}Fisher's exact test (two sided);
^{c}ER neg, score = 0; ER pos, score =12 ;
^{d}PR low, 0 ≤ score ≤ 6; high, 6 < score ≤ 12; PR, progesterone receptor;
^{e}Her2/erbb2, low, score 0 or 1; high, score 2 or 3.

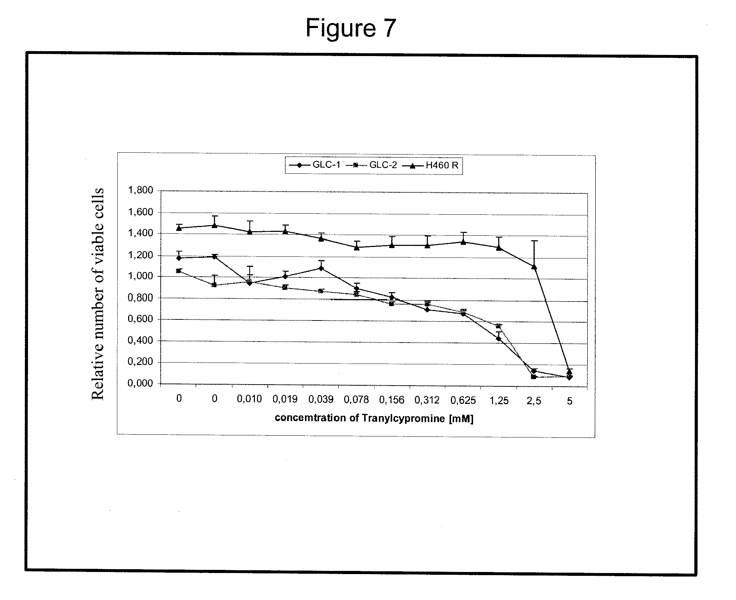

### 8/9: Fig. 7: Reduction in cell growth in three different lung carcinoma cell lines upon MAOI treatment.

Three different lung carcinoma cell lines (GLC-1, GLC-2, H460 R) were treated with tranylcypromine at the indicated concentrations for 72 h, followed by an MTT assay in order to determine the amount of viable cells (expressed in relative numbers on the y-axis).

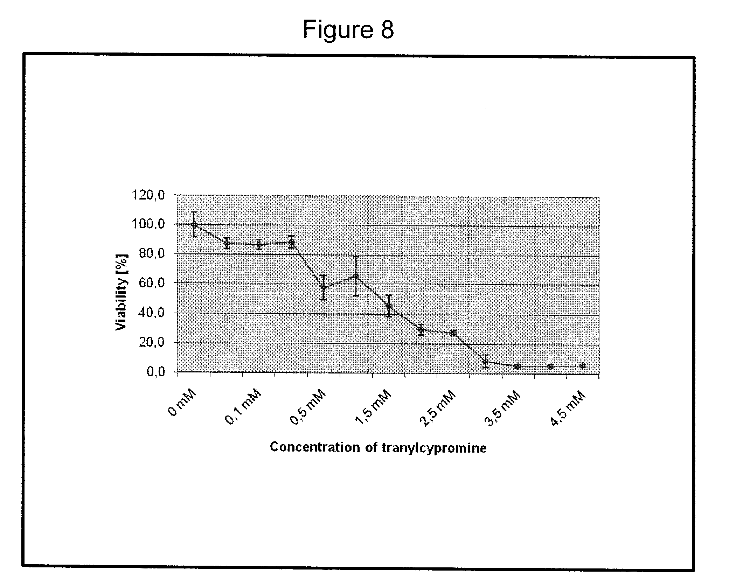

### 9/9: Fig. 8: Reduction in cell growth in the sarcoma cell line 1273 upon MAOI treatment.

Cells of the above mentioned sarcoma cell line were treated with tranylcypromine at the indicated concentrations for 72 h, followed by an MTT assay in order to determine the amount of viable cells (expressed as % viability on the y-axis).

### DETAILED DESCRIPTION OF THE INVENTION

As already set out above, the present invention partially resides in the surprising finding that the expression level of lysine specific demethylase 1 (LSD1) can be used as biomarker in breast cancer and that the inhibition of LSD1 results in growth arrest of tumor cells selected from breast cancer cells, lung carcinoma cells and sarcoma cells; thus, inhibiting LSD1 may be used as therapeutic approach of treating said cancers.

### 1. Definitions

Before some of the embodiments of the present invention are described in more detail, the following definitions are introduced.

As used in the specification and the claims, the singular forms of "a" and "an" also include the corresponding plurals unless the context clearly dictates otherwise.

The terms "about" and "approximately" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of" is considered to be a preferred embodiment of the term "comprising of". If hereinafter a group is defined to comprise at least a certain number of embodiments, this is also meant to encompass a group which preferably consists of these embodiments only.

The term "cancer" and subtypes of cancer, such as "breast cancer", "lung carcinoma" and "sarcoma" are used herein according to the general definitions in the medical field.

The term "diagnosing breast cancer" is used herein in its common meaning as to answer the question whether a subject suffers from breast cancer or not. Diagnosing breast cancer indicates that the person indeed suffers from breast cancer.

The term "substantiating the diagnosis of breast cancer" as used herein is meant to describe a situation, wherein the subject has already been diagnosed with breast cancer. However, due to the fact that a diagnosis can also include false-positive results and may vary from individual to individual, the determination of additional breast cancer indicating factors can be used in order to substantiate and thus strengthen said diagnosis.

The term "subject" as used herein preferably indicates a human being, more preferably a female human being, but may also refer to animals.

The term "breast tissue" as used herein relates to cellular and non-cellular components, which together form the breast tissue as part of the body. However, it is preferred that breast tissue comprising cells is provided in step a) of the method of the present invention. Breast tissue may comprise cells of different states of differentiation within one lineage of cells and/or cells differentiated in different lineages in order to fulfill specific functions within the breast tissue.

The term "sample of breast tissue" is meant to describe a certain amount of breast tissue, given e.g. in mg-amounts, wherein said breast tissue preferably comprises an agglomeration of cells. Thus, said sample may comprise cells of different origin within breast tissue or may be taken from one kind of differentiated cells only.

The term "expression level" as used herein may, as already pointed out above, not only refer to the LSD1 gene, but also e.g. to the LSD1 mRNA or the LSD1 protein. In this regard, the expression level may be expressed differently, such as e.g. in terms of the amount or concentration of the LSD1 species analyzed and is always normalized to a parameter (such as e.g. the total amount of the species analyzed). Thus, if the LSD 1 protein is analyzed, normalization may be carried out using the overall protein amount in the sample. Accordingly, analyzing the LSD mRNA, the overall mRNA amount in the sample may be used for normalization and so on. Obviously, the overall weight of a sample analyzed may also be used for normalization as well as the volume of the sample.

In the following, a brief overview of the concept of gene expression with focus on LSD1 will be given.

One may refer to the LSD1 protein as the final product of LSD1 gene expression. "LSD1" or "lysine specific demethylase 1" as used herein refers to the previously identified LSD1 (see introduction above), wherein the amino acid sequence of LSD1 is given in SEQ ID No. 5. Thus, the expression level of the LSD1 protein may be determined in a sample.

Since posttranslational modifications of the LSD1 protein, such as phosphorylation and the like as stated above, may have an influence on the LSD1 activity, such modifications may also be analyzed.

The cellular process for generating the LSD1 protein is the translation of the corresponding LSD1 mRNA. Therefore, the expression level may also be expressed as LSD1 mRNA levels. As outlined above, the sample may thus be analyzed for its mRNA amount.

The step preceding mRNA translation resides in the generation of mRNA from the LSD1 gene, the transcription of said gene. Since there may be a correlation between gene copy numbers of a given gene and the expression level of said given gene, one may also analyze the number of gene copies of LSD1 present in the genomic material, the so-called "copy numbers" of the LSD1-gene. In case said gene should be present in more than one copy, one could expect an overall higher LSD1 expression in said sample.

However, since gene expression is regulated by specific factors in a cell, one may also analyze said regulatory factors in order to determine whether a gene will be aberrantly expressed due to misregulation. So-called "promoter regions" regulating gene expression are present either 5' to the coding DNA or in DNA regions further apart. It seems that negative and positive factors influencing gene expression assemble on said promoters and regulate and/or fine tune gene expression.

Thus, one may also analyze the promoter regions regulating LSD1 expression in order to find out whether an increase or decrease of gene expression can be expected. Point mutations, deletion, insertions and the like on the DNA promoter regions as well as epigenetic marks as mentioned above represent targets, which may be analyzed in this regard.

In summary, the term "expression level" may thus preferably be used according to the present invention either with respect to the copy number of the LSD 1 gene, the amount of corresponding mRNA as well as the amount of LSD1-protein.

The term "reference" as used herein refers to an analysis of a sample of a healthy subject, i.e. a subject not suffering from breast cancer.

The term "course of breast cancer" is used herein with respect to the prognosis of breast cancer in the subject and/or the prediction of events linked to said breast cancer. Different events are typically observed during the course of breast cancer and may thus be predicted during the further course of said cancer, such as e.g. the growth rate, the invasion and/or the process of metastasizing. One may also determine the overall course of the breast cancer, denoted as survival probability.

The term "aggressive biology of breast cancer" is linked to the development of a cancer over time and thus indirectly also to the term "prognosis" as explained above; thus, a particular aggressive biology of a cancer may manifest in rapid growth of the tumor and/or rapid invasion of other tissues and/or metastasis of the tumor in different areas of the body. Generally, there is a strong link between the aggressiveness of a tumor and the survival chance.

The term "solution" as used with regard to the third object mentioned above refers to a buffer, which is routinely used in laboratories when analyzing proteins. Preferably, said buffer is selected from a phosphate-buffered saline, Tris-buffer or the like, but also from common lysis-buffers comprising detergents and the like. In preferred embodiments, the buffer does not induce complete denaturation of the proteins.

The term "coating" as used herein refers to the process of covalently binding proteins to a surface, e.g. via coupling to a reactive surface. Preferably, said process is not protein-specific and results in a surface of protein displaying accessible epitopes. Preferably, said proteins are prior and subsequently to the coating in a solution as outlined above or alternatively in a solution adapted to the coupling reaction. Such coupling reactions as well as corresponding kits are routine laboratory equipment and known to the skilled person.

A "well" as used herein is meant to describe a means displaying several surfaces, wherein one surface is preferably surrounded by other surfaces such that said first surface may be completely encompassed by e.g. a solution. An example for a typical well is a well of a 96-well plate known to the skilled person.

A "compound specifically binding to said LSD1 antibody" in the meaning of the present invention preferably relates to a so-called secondary antibody, wherein the variable region of said secondary antibody preferably recognizes the constant Fc part of the LSD1-antibody used in the first incubation step. Said compound is furthermore preferably coupled to either a dye, such as a fluorescent or luminescent dye, or an enzyme, such as horseradish-peroxidase or the like, such that said compound is able to excite a signal upon stimulation of said second component.

"Stimulation" said compound may accordingly done by either e.g. exciting the fluorescence or luminescent part, or by incubating said compound with a substrate of the enzyme component such that a product is generated, which can be detected (a "signal"), e.g. a specific color created upon turnover of the substrate into product.

"Quantifying" as used herein refers to the process of quantitatively detecting the signal mentioned above; this is preferably done using a regular ELISA-reader or any other device suitable for said quantification.

Finally, the "assignment" of the signal intensity to a specific amount or concentration of LSD1 protein, which is done according to a standard curve as described above, is preferably done by using automated means, such as software.

### 2. General description of the methods of the invention

Regarding the first aspect of the present invention, the whole process of diagnosis and/or substantiating a diagnosis may start with a mammography or the like. Thus, a subject's breast may be analyzed for cell tissue suspicious of being a tumor. At this stage, a sample of the above-mentioned suspicious tissue may be gained, e.g. by a biopsy.

When diagnosing breast cancer based on the expression level of LSD1, said sample is then provided according to step a) of the first object of the present invention and treated according to said method. However, said method may also be used to substantiate the diagnosis of breast cancer; thus, the sample may have been analyzed prior to being treated according to the method of the present invention, e.g. for the expression of estrogen receptor and/or progesterone receptor and/or HER2-expression or according to the mammography. In this alternative case, the determination of the expression level of LSD1 according to the first object of the present invention may then be used to substantiate the diagnosis of breast cancer, which was based on different markers than LSD1.

According to the second object of the present invention, said method may also be used for determining the course of breast cancer in a subject suffering from breast cancer. In this object, the diagnosis of breast cancer has already been provided using the first object of the invention and/or different methods. Therefore, the first and second object of the present invention may also be applied in combination. If, in this second method of the invention, a particularly high expression level of LSD 1 can be observed, this is indicative of an aggressive biology of said breast cancer. This is due to the correlation surprisingly found by the inventors, namely that the expression level of LSD1 correlates with an aggressive biology of said tumor.

In all three cases as outlined in the above paragraphs, the expression level of LSD1 needs to be determined.

As already discussed above, the expression level may be determined using either the gene copy number and/or the amount of LSD1 mRNA and/or the amount of LSD1-protein.

According to which method is chosen, different analysis methods are available.

Thus, for the analysis of the gene copy number of LSD1, one may use an assay, wherein probes specific for the LSD1 genomic DNA are used, which hybridize in a quantitative way with genomic DNA and are labeled such that they may be quantified. Furthermore, quantitative sequencing methods or the like may also be used. In general, a higher copy number than the regular copy number of LSD1 may be indicative of breast cancer. Thus, a copy number of 2, 3, 4 or even 5 genes of LSD1 present in the genomic DNA of a subject can be indicative for a higher expression level than usual and thus for breast cancer.

Second, the mRNA level may be determined; to this end, the skilled person is aware of several methods, such as real-time-PCR, reverse transcription into DNA followed by regular quantitative PCR in order to quantify the cDNA, quantitative Northern-blot analysis using e.g. radiolabeled probes specific for LSD1 mRNA.

Another way of determining the expression level is by analyzing the protein amount of LSD1 in a sample. To this end, the skilled person is also aware of several methods such as methods selected from an ELISA-assay, Western-blot analysis, mass spectrometry, Immunostaining and Immunoprecipitation.

For all above-mentioned methods, a normalization step as mentioned above needs to be carried out.

Furthermore, not only the expression level of LSD1 in the sample to be analyzed needs to be determined, but also the expression level of LSD1 in a sample derived from a healthy subject needs to be known.

In one embodiment, a sample from a healthy patient is analyzed according to the analysis of the sample of the subject in question, i.e. the determination of the LSD expression level is carried out exactly as in the subject to be analyzed.

However, in a preferred embodiment, said expression levels are already known from a large group of reference subjects, wherein said subjects are healthy subjects. Thus, samples of said large reference group were analyzed for the expression level of LSD1, are known and can accordingly be taken from a database.

Again, it should be emphasized that also the expression levels in the control subjects need to be normalized in order to being able to compare them with the expression levels in different samples. Thus, also in the control analysis, the amount of gene copies, the amount of mRNA or the amount of LSD1-protein needs to be normalized to e.g. the standard copy number, the amount of total mRNA in the sample or the amount of total protein in the sample.

Particularly for the analysis of a specific protein, a well established, well-known and relatively simple method for analysis of the protein amount or concentration is an ELISA-assay. The main steps of such an assay were already mentioned above; thus, the general scheme will only be outlined briefly in the following with respect to LSD1, as one object of the present invention.

First, a sample of tissue comprising cells comprising proteins is provided. This may be a sample of breast tissue; however, other tissue may be used as well. Preferably, said sample is provided outside the human or animal body. The sample may have been gained as mentioned above, e.g. by a surgery or biopsy.

Alternatively, cells cultured in vitro may also be used as sample. Of course, recombinantly expressed LSD1 may also be analyzed for its amount and/or concentration.

In a second step, said sample comprising cells is, if necessary, lysed such that a protein lysate is provided; thus, the proteins are now present in a solution, preferably without any cell debris or the like, such as cell walls and/or DNA or the like. This may be achieved by high and/or low speed centrifugation steps, optionally combined with specific detergents. Preferably, said protein lysate is prepared at 4° such that the proteins are in their native condition. Preferably, the coating may also be carried out at 4°C, whereas all subsequent binding and washing steps may be carried out at room temperature. Different protocols for lysing cells are known to the skilled person and can be used in the present invention as long as they do not result in proteins denatured to such an extent that they cannot be recognized by antibodies any more. A typical lysis protocol is comprised of the following steps:
- collecting the cells to be analyzed;
- resuspending and optionally washing the cells in washing buffer;
- resuspending the cells in lysis buffer, wherein said lysis buffer may comprise detergents in order to break the cells open. Alternative lysis methods comprise pressure (French press), sonification or the like;
- spinning the lysate in order to remove cell debris and collecting the supernatant comprising proteins.

After a lysate comprising proteins has been obtained, said proteins are coupled to a surface of a well as mentioned above, e.g. to the bottom surface of a 96-well.
However, other wells may of course also be used according to the invention. To this end, a concentration or dilution step may be necessary in order to use concentrations of the protein lysate as mentioned above. The coating step may be done according to standard protocols, such as e.g. the protocol by Nunc (Wiesbaden, Germany) using coating buffer and Maxisorb microplates. This may include an overnight incubation and washing steps.

After coating, said proteins are then incubated either with an LSD1-antibody or a fragment of an LSD1-antibody. Alternatively, a conjugate comprising an LSD1-antibody or fragment thereof and at least one further component may be used. "Fragment" in this regard refers e.g. to a single chain antibody, a truncated LSD1-antibody or the like. In summary, this first compound is referred to as the "first antibody". When using the LSD1-antibody by Novus Biologicals, said antibody is preferably used in a dilution of about 1:400, but may also be used in different dilutions according to the sample analyzed. In this regard, testing different dilutions of an antibody is within the knowledge of a skilled person. Thus, in this first step, LSD1 proteins coupled to the surface are specifically recognized and bound by the first antibody diluted in appropriate buffer, such as blocking buffer (e.g. PBS-T).

In order to remove unspecifically bound antibodies, fragments or conjugates thereof, the wells are preferably washed with optionally differently stringent buffers (e.g. PBS-T solution comprising different Tween concentrations) for several times.

Subsequently, a second reagent or "second antibody" is added to the complex. If an LSD1-antibody is used as first antibody, the second reagent is preferably an antibody directed to the LSD1-antibody. Thus, a second immuno-reactive binding is achieved, and a complex comprising the LSD1 protein, the LSD1-antibody as well as the second reagent is formed. As already outlined above, said second antibody may be coupled to a component, wherein said component and/or products processed by said components are then detected in the detection step and quantified.

Alternatively, LSD1-antibody conjugates may also be used. In this case, there is no second compound detecting the LSD1-antibody or fragment thereof, but rather the first LSD1-antibody is already coupled to a compound, which may subsequently be detected and quantified. Such a compound may again be a dye such as a fluorescent dye, or an enzyme, such as horseradish peroxidase or the like.

In any case, the "signal" as defined above is then detected quantitatively, e.g. by determination of the optical density at a wavelength corresponding to the product. Alternatively, when using fluorescence or luminescent components, said components may be excited and the corresponding emission may be recorded.

In any case, after having quantitatively detected the "second compound", the amount of LSD1 protein in the sample can be assigned according to a standard curve. It should be emphasized, however, that such an analysis may be performed only within a linear range of protein amounts. The present standard curve using recombinantly expressed LSD1 for determining this linear range is in this regard applicable for all analyses directed to the determination of the amount of LSD1.

As mentioned above, the inventors have not only found that LSD1 can be used as biomarker for breast cancer, but that the inhibition of LSD1 also represents an effective means for treating cancer. LSD1 seems to be implicated in the activation of cell cycle promoting genes and thus the aberrant growth of cells, and thus represents a very interesting new target for cancer therapy. Since LSD1 activity seems to promote transformation of cells, the inhibition of the enzymatic activity of LSD1 seems to result in turn in the inhibition of cell growth and malignancy.

LSD1 likely demethylates methylated histones by its monoamine oxidase domain. Other demethlyases such as JHDM1 and JHDM2 rely on other mechanisms (Lan et al., Current Opinion in Cell Biology, 20, 316-325 (2008). Other enzymes having a monoamine oxidase domain such as LSD1 are Monoamine oxidase A (MaoA) and Monoamine oxidase B (MaoB) (Shih et al., Annu. Rev. Neurosci., 22, 197-217 (1999). Therefore, MAO inhibitors can also be used to inhibit LSD1.

Many of said MAO inhibitors are already well characterized, also in terms of their general pharmaceutical characteristics. However, "pharmaceutical compositions" as used herein, will briefly be explained in the following.

"Pharmaceutically active agent" as used herein means that a compound is potent of modulating a response in a human or animal being *in vivo.* When reference is made to a compound as "the only pharmaceutically active agent", this is meant to describe that the activity of a corresponding pharmaceutical composition is due to said active agent only.

The term "pharmaceutically acceptable excipient" as used herein refers to compounds commonly comprised in pharmaceutical compositions, which are known to the skilled person. Such compounds or excipients are exemplary listed below.

In view of the definition "pharmaceutically active agent" as given above, a pharmaceutically acceptable excipient may thus be defined as being pharmaceutically inactive.

A pharmaceutical composition may be formulated for oral, buccal, nasal, rectal, topical or parenteral application. Parenteral application may include intravenous, intramuscular or subcutaneous administration. An LSD1 inhibitor may be applied in pharmaceutically effective amounts, for example in the amount set out herein below.

The pharmaceutical compositions of the invention may be made from the compounds of the invention alone or they may comprise pharmaceutically acceptable excipients. Such compositions may also be designated as formulations.

Pharmaceutical dosage forms may be solid or liquid dosage forms or may have an intermediate, e.g. gel-like character depending *inter alia* on the route of administration.

In general, the inventive dosage forms will comprise various pharmaceutically acceptable excipients which will be selected depending on which functionality is to be achieved for the dosage form.

A "pharmaceutically acceptable excipient" in the meaning of the present invention can be any substance used for the preparation of pharmaceutical dosage forms, including but not limited to coating materials, film-forming materials, fillers, disintegrating agents, release-modifying materials, carrier materials, diluents, binding agents and other adjuvants. The term carrier denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

As regards human patients, the LSD inhibitor may be administered to a subject in an amount of less than 1 µM.

### 3. Examples

In the following, examples of embodiments of the present invention are outlined. However, said examples should not be construed as limiting the scope of the present invention.

### 3.1. Materials and Methods

### Tissue specimens

Paraffin-embedded tissue specimens and frozen tissue were selected from the archival files of the Institute of Pathology, University of Bonn. Some of the tissue specimens were immediately kept frozen after resection and stored in liquid nitrogen until further use. Tissue processing of all specimens was done using identical procedures. Clinicopathological variables measured at diagnosis were obtained from patient records. The study adheres to ethical standards and was approved by the ethics committee (36/08).

### Immunohistochemistry

Immunohistochemical staining was done as previously described [Schulte, J.H. et al. (2009) Lysine-specific demethylase 1 is strongly expressed in poorly differentiated neuroblastoma: implications for therapy. Cancer Res., 69, 2065-20719], using an α-LSD1 antibody (Cat. No. 100-1762, Novus Biologicals, Littleton, USA) diluted 1:250. Nuclear immunostaining results for LSD1 were evaluated using a semiquantitative Remmele scoring system [Remmele, W. et al. (1986) Comparative histological, histochemical, immunohistochemical and biochemical studies on oestrogen receptors, lectin receptors, and Barr bodies in human breast cancer. Virchows. Arch. A. Pathol. Anat. Histopathol., 409, 127-147], calculating the staining intensity and the percentage of positive cells. Briefly, the number and intensity of positive cells were counted and scored between 0 and 4 (0 = no positive nuclei, 1 = < 10% nuclei display intense staining or more nuclei display weak staining, 2 = 11-50% intense staining, or more nuclei display moderate staining, 3 = 51-80% nuclei display intensive staining, 4 = 81-100% nuclei display intensive staining). Scoring procedures and controls were described previously [Kahl, P. et al. (2006) Androgen receptor coactivators lysine-specific histone demethylase 1 and four and a half LIM domain protein 2 predict risk of prostate cancer recurrence. Cancer Res., 66, 11341-11347].

### ELISA

For ELISA analysis, 20 normal breast tissues, 26 ER-positive and 37 ER-negative breast tumor tissues were used. Hematoxylin-eosin-stained sections were prepared for assessment of the percentage of tumor cells; only samples with >70% tumor cells were selected. 96-well Maxisorb microplates (Nunc, Wiesbaden, Germany) were incubated with 100 µl tissue protein lysates (corresponding to 40 µg, in coating buffer: 50 mM sodium carbonate buffer, pH 9.2) overnight at 4 °C. After removal of the coating solution by inverting the plate, the wells were blocked with 200 µl blocking buffer (Roche, Mannheim, Germany) for 1 h at room temperature. After rinsing with washing buffer (0.05 % Tween in PBS, PBS-T), the wells were incubated with α-LSD1 solution (1: 400, Novus Biologicals, Cat. No NB 100-1762) in 100 µl blocking buffer for 1 h at 25 °C followed by three washing steps with 200 µl PBS-T. After addition of 100 µl HRP-labelled α-mouse (1:1000, DAKO, Glostrup, Denmark, Cat. No. P-0448), the wells were incubated for 0.5 h and washed three times. Finally, 100 µl of the TMB substrate solution (1 Step Ultra TMB, Thermo Scientific, Rockford, USA) were added to each well. The conversion of substrate was stopped by addition of 100 µl of 2 N sulphuric acid solution. The optical density was determined in an ELISA reader (ELX 800 Universal, Biotek Instruments, Winooski, USA) at 450 nm.

### Cell culture and proliferation assays

MCF7, MDA-MB 453 and MDA-MB 231 breast cancer cells were cultivated in DMEM, T47D breast cancer cells were cultivated in RPMI, GLC-1, GLC-2 and H460 R lung carcinoma cells were cultivated in IMDM, and sarcoma cells 1273 were cultivated in Häm's F12. All media were supplemented with 10% FCS, L-glutamine and antibiotics.

To measure cell growth cells were seeded at a density of 2500 cells per well in 96 well microplates, and cultured in standard medium. Treatment with clorgyline (Sigma-Aldrich, Hamburg, Germany) or tranylcypromine (Biomol, Hamburg, Germany) was done as indicated. An 3-(3,4-dimethylthiazol-2-yl)-2,5-diphenyltetrazolium bromide (MTT) assay was performed according to the manufacturer's protocol (Roche, Mannheim, Germany).

### RNA Isolation, RT-PCR and quantitative RT-PCR (qRT-PCR)

Total RNA was isolated from cells using the RNeasyMini kit (Qiagen, Hilden, Germany), and cDNA synthesis was performed using the SuperScript Reverse Transcription kit (Invitrogen, Karlsruhe, Germany).

Gene expression was monitored by quantitative real-time PCR (Applied Biosystems, Foster City, USA). Expression values were normalized to the mean of 18s rRNA.

### Western blot analysis

Protein lysates were extracted from cells and blotted as described in Schulte *et al.* [Schulte, J.H. *et al.* (2009), see above] The membranes were incubated for 1-2 hours using the following antibodies and dilutions: α-LSD (Novus Biologicals) 1:1000; α-K4H3me2 (Abcam, Cambridge, UK) 1:1000; β-actin (Sigma-Aldrich, Hamburg, Germany) 1:5000; Her2/erbB2 (DAKO), 1:1000. Coomassie staining was used as a loading control, since the frequently used reference protein β-actin was clearly up-regulated in the cancer specimens [Goidin, D. et al. (2001) Ribosomal 18S RNA prevails over glyceraldehyde-3-phosphate dehydrogenase and beta-actin genes as internal standard for quantitative comparison of mRNA levels in invasive and noninvasive human melanoma cell subpopulations. Anal. Biochem., 295, 17-21].

### SiRNA transfection

Cells were seeded with 1x10⁵ cells in 24 well plates, then incubated for 3 - 12 days in standard medium in the presence of 10-20 nM siRNA directed against LSD1 (targeted on exon 8; Ambion, Austin, USA) or control siRNA (scrambled) complexed with HiPerFect Transfection Reagent (Qiagen) according to the manufacturer's instructions.

### Chromatin immunoprecipitation (ChIP)

ChIP experiments were performed essentially as described in Schulte *et al.* [Schulte, J.H. *et al.* (2009), see above]. MCF7 cells were transfected 3 days before harvesting for ChIP with or without LSD1 siRNA (Ambion) following the manufacturer's instructions. Immunoprecipitation was performed with specific antibodies to H3K4me2 (Abcam), H3K9me2 (Abcam) and LSD1 (Novus Biologicals) on protein A coupled Dynabeads (Invitrogen). Purified DNA specimens were subjected to real-time PCR using a SYBR green probe (Invitrogen) in an ABI Prism 7900 (Applied Biosystems), according to the manufacturer's specified parameters. Amplicons were normalized to the DNA immunoprecipitated with antibody to histone H3 (Abcam). The following TaqMan real-time PCR primers were used for the *CCNA2* (-137 to - 30) proximal promoter region: forward primer, 5'-CCTGCTCAGTTTCCTTTGGT-3' (SEQ ID No. 1); reverse primer, 5'-ATCCCGCGACTATTGAAATG-3' (SEQ ID No. 2). The following primers were used to detect *ERBB2* (-309 to -220) proximal promoter region: forward primer, 5'-GGCTTGGGATGGAGTAGGAT-3' (SEQ ID No. 3); reverse primer, 5'-TCCCTAGGCTGCCACTCTTA-3' (SEQ ID No. 4).

### Statistical analysis

Statistical significance of the ELISA results was tested by two-sided, non-parametrical Mann-Whitney U-test to analyze differences in protein levels among distinct groups using SPSS 17.0 program (SPSS, Inc., Zürich, Switzerland). Association between categorical variables was assessed by two-sided Fisher's exact test using GraphPad Prism 5 (La Jolla, USA).

### Cloning, expression and purification of LSD1

LSD1ΔN (166-852) was subcloned into the pET15B vector. N-terminal His₆-tagged LSD1 was expressed in TB in *Escherichia coli* BL21 (DE3 Star). Protein production was induced by 0.75 - 1 mM IPTG overnight at 20°C. Harvested cell were resuspended in lysis buffer containing 2xPBS and 20 mM imidazole. The lysate was applied to Ni²⁺ affinity column and His₆-tagged LSD1 ΔN was eluted with elution buffer (100 mM Tris-HCl pH 8.0, 300 mM imidazole). The protein was concentrated and then purified on a Superdex 200 (16/60) column (GE Healthcare, Piscataway, U.S.A.) in 20 mM Tris-HCl pH 8.0, 100 mM NaCl. His₆-tagged LSD 1 containing fractions were pooled and applied to a HitrapQ ion exchange column (GE Healthcare, Piscataway, U.S.A.) and eluted with a buffer containing 50 mM Tris-HCl ph 8.0. Sample purity was checked by SDS-PAGE and protein concentration was determined by Bradford assay using BSA as the standard. Purified LSD1 was stored in 20 mM Tris-HCl pH 8.0, 100 mM NaCl, 5 % Glycerol.

### Development of LSD1 ELISA

Microplates were incubated with a series of dilution of recombinant His₆-tagged LSD1ΔN protein (1.0, 3.1, 9.3, 27.8, 83.3 and 250.0 µg/L) and breast tumor tissue protein lysate (3, 10, 30 and 60 µg/well) in 100 µl coating buffer (50 mM sodium carbonate buffer, pH 9.2) overnight at 4 °C. Antibody dilution ratio was modified for the recombinant LSD1. (α-LSD1, 1:4000; HRP-labelled α-mouse, 1:1000). Otherwise, all steps were performed as described above.

### 3.2 Results

### LSD1 is strongly expressed in ER-negative breast cancer

For this study, LSD1 expression both in fresh-frozen and in formalin-fixed, paraffin-embedded tissue specimens of ductal and lobular breast cancer was retrospectively analysed. Initial immunohistochemical staining revealed moderate nuclear expression in luminal cells of normal breast glands and ER-positive cancers (histological grade 2). Significantly more intense staining was observed in ER-negative breast cancers (histological grade 3), in which every tumor cell showed a strong and specific nuclear staining pattern (Figure 1A).

Therefore, the LSD1 expression levels were measured by a quantitative LSD1 ELISA. The assay was validated by recombinant LSD 1 protein and performed in a quantitative manner over a broad spectrum of LSD1 protein concentrations between 1 to 250 µg/l and also after serial dilution of protein lysates from breast cancer tissue specimens (Figure 5). In protein lysates of snap-frozen primary breast tissues including 20 normal breast tissues, 26 ER-positive and 37 ER-negative breast tumors, LSD1 protein was significantly stronger expressed in ER-negative breast cancers than in ER-positive cancers or normal tissue (Mann-Whitney-U-test, *P* < 0.001, Figure 1B and 1D). There was a trend of slightly higher expression comparing ER-positive breast cancer and normal breast tissue. Similar results were seen in a small set of breast cancer specimens analyzed by Western blot analysis (Figure 1 C, N indicates normal breast tissue).

Significant inverse correlation between LSD1 expression and ER status was also seen in detailed immunohistochemical analysis. To statistically calculate the association between histopathological parameters and LSD1 expression levels (Figure 6), tumor specimens were classified into a group with low LSD1 expression (n=16) and a second group with high LSD1 expression (n=22). Results in Figure 6 clearly indicate that strong nuclear LSD1 staining (score > 9) was associated with negative ER status (score = 0) (Fisher's exact test, *P* < 0.001, Figure 6). Consistently high LSD1 expression also correlated with low PR expression (score ≤ 6) (*P* = 0.001).

Considering that hormone receptor expression in breast cancer is associated with a significantly better prognosis [Cui, X. et al. (2005) Biology of progesterone receptor loss in breast cancer and its implications for endocrine therapy. J. Clin. Oncol., 23, 7721-7735], high LSD1 expression appears to provide a biomarker for aggressive tumor biology associated with hormone receptor-negative breast cancer.

### LSD1 inhibition using monoaminoxidase inhibitors (MAOIs) confers growth inhibition and increase of global H3K4 methylation in breast cancer cell lines

The catalytic domains of LSD1 and monoaminoxidases (MAOs) share structural homology and make use of the same catalytic mechanism [Lee, M.G. et al. (2006) Histone H3 lysine 4 demethylation is a target of nonselective antidepressive medications. Chem. Biol., 13, 563-567]. Therefore, the MAO inhibitors tranylcypromine and clorgyline were used to inhibit LSD1 in breast cancer cell lines *in vitro.* Four different breast cancer cell lines, all of which strongly expressed LSD1 (Figure 2B) were tested. Treatment with tranylcypromine and clorgyline for 72 hours impaired cell growth in a dose-dependent manner (Figure 2A) in all four cell lines. To address whether reduced cell viability after treatment with MAOIs correlates with LSD1 inhibition, the methylation status of lysine 4 in histone 3 in cells before and after treatment was analyzed. Upon treatment of MAOIs, global di-methylation of lysine 4 in histone H3 increased, while LSD1 enzyme levels were not altered (Figure 2B).

### LSD1 inhibition using monoaminoxidase inhibitors (MAOIs) confers growth inhibition in lung carcinoma and sarcoma cancer cell lines

The MAO inhibitor tranylcypromine was also used to inhibit LSD1 in three lung carcinoma cell lines and one sarcoma cancer cell line *in vitro.* Treatment with tranylcypromine for 72 hours impaired cell growth in all cell lines tested, as indicated in Figures 7 and 8.

### siRNA mediated knock-down of LSD1 reduces cellular growth

To analyze the consequences of reduced LSD1 expression, MDA-MB 453 and MDA-MB 231 cells were transiently transfected with 15 nM siRNA directed against LSD1 or with 15 nM scrambled control siRNA. Significant LSD1 knock-down was detected measuring protein levels 3 days after transfection by Western blot (Figure 3B). MTT assays indicated that the silencing of LSD1 caused a significant decrease in cell growth and viability (Figure 3A). Similar effects were also seen in MCF7 and T47D cells. Morphologically no sign of apoptosis was detected, but the LSD1 inhibition appeared to affect the number of dividing cells consistent with a previous report that inhibition of LSD1 leads to G2/M cell cycle arrest [Scoumanne, A. et al. (2007) The lysine-specific demethylase 1 is required for cell proliferation in both p53-dependent and -independent manners. J. Biol. Chem., 282, 15471-15475].

### Knock-down of LSD1 induces downregulation of proliferation associated genes and alters gene-specific H3K9 methylation

Considering that LSD1 regulates gene-expression through modification of histone methylation in gene promoter regions and previous evidence that silencing of LSD1 decreased cellular proliferation, the expression of proliferation related genes by quantitative RT-PCR (qRT-PCR) was further analyzed. As illustrated in Figure 4A *CCNA2* and *ERBB2* were downregulated after LSD1 knock-down both in ER-positive MCF7 or ER-negative MDA-MB 231 and MDA-MB 453 cells. Down-regulation of the Her2/erbB2 protein level was confirmed 6 days after knock-down of LSD1 in MDA-MB 231 cells (Figure 4B).

To asses whether the promoters of *CCCNA2* and *ERBB2* are direct or rather indirect targets of histone modification by LSD1, MCF7 cells treated with siRNA directed against LSD1 or with a scrambled control siRNA were subjected to chromatin immunoprecipitation (ChIP) using α-LSD1, α-K9H3me2 and α-K4H3me2 antibodies. ChIP analysis indeed confirmed that LSD1 is present at the proximal promoter of the *CCNA2* and *ERBB2* gene. Knock down of LSD1 decreased the occupancy of LSD1 on *CCNA2* (from -137 to -30) and *ERBB2* (from -309 to -220) promoter regions (Figure 4C, left panel). This was accompanied by significant increase in dimethylation on H3K9 which has been previously shown to result in transcriptional repression (Figure 4C, middle panel). In contrast, after LSD1 knock-down, genomic DNA corresponding *CCNA2* and *ERBB2* proximal locus were not enriched with α-H3K4me2 antibody (Figure 4C, right panel). This finding is consistent with results observed above for down-regulation of *ERBB2* and *CCNA2* upon knock-down of LSD1, suggesting that LSD1 regulates directly the transcription of *CCNA2* and *ERBB2* through demethylation of H3K9, but not through demethylation of H3K4.

## Claims

1. Method of diagnosing breast cancer and/or substantiating the diagnosis of breast cancer in a subject comprising at least the steps of:
a) Providing a sample of breast tissue of said subject outside the human or animal body;
b) Determining the expression level n1 of lysine specific demethylase 1 (LSD1) in said sample;
c) Comparing the expression level n1 obtained in step b) to a reference expression level n2 of LSD1 in a sample of breast tissue of a healthy subject;
d) Diagnosing breast cancer and/or substantiating the diagnosis of breast cancer based on said comparison, wherein n1 > n2 is indicative of breast cancer.

2. Method of determining the course of breast cancer in a subject comprising at least the steps of:
a) Providing a sample of breast tissue of said subject outside the human or animal body;
b) Determining the expression level n1 of lysine specific demethylase 1 (LSD1) in said sample;
c) Comparing the expression level n1 obtained in step b) to a reference expression level n2 of LSD1 in a sample of breast tissue of a healthy subject;
d) Determining the course of breast cancer based on said comparison, wherein n1 > n2 is indicative of an aggressive biology of said breast cancer.

3. Method according to claim 2, wherein the aggressive biology of said breast cancer correlates with n1, i.e. the higher n1, the more aggressive the breast cancer.

4. Method according to any of the preceding claims, wherein the expression level of LSD1 is determined by analyzing the LSD1 gene and/or by analyzing the mRNA transcribed from the LSD1 gene and/or by analyzing the LSD1 protein.

5. Method according to claim 4, wherein the LSD1 gene is analyzed for its gene copy number using a method selected from the group of methods comprising quantitative hybridization techniques using oligonucleotides directed to the LSD1 gene, quantitative sequencing analysis and combinations thereof.

6. Method according to claim 4, wherein the mRNA transcribed from the LSD1 gene is analyzed for its amount using a method selected from the group of methods comprising quantitative PCR, real-time-PCR, Northern blot analysis and combinations thereof.

7. Method according to claim 4, wherein the LSD1 protein is analyzed for its amount using methods selected from the group of methods comprising an ELISA-assay, Western-blot analysis, mass spectrometry, Immunostaining, Immunoprecipitation, chromatography and combinations thereof.

8. Method of determining the amount and/or concentration of the LSD1 protein in a sample, wherein said method comprises at least the following steps:
a) Providing a solution of said sample comprising proteins, wherein said proteins are present in a concentration of between about 0.01 mg/ml and about 10 mg/ml, preferably between about 0.05 mg/ml and about 5 mg/ml, more preferably between about 0.1 mg/ml and about 1 mg/ml and most preferably of about 0.4 mg/ml or 0.5 mg/ml in said solution;
b) Coating said proteins onto a surface of a well;
c) Incubating said well with a solution comprising an LSD1-antibody;
d) Incubating said well with a solution comprising a compound specifically binding to said LSD1-antibody, wherein said compound excites a signal upon stimulation;
e) Stimulating said compound;
f) Quantifying the signal excited upon stimulation; and
g) Assigning an amount and/or a concentration to the LSD1 protein in said sample according to the intensity of said signal using a standard curve.

9. Method according to claim 8, wherein said standard curve was recorded using recombinant LSD1 and is linear over an amount of LSD1 ranging from about 2.5 ng to about 25 ng corresponding to a concentration range of about 0.025 µg/ml to about 0.25 µg/ml.

10. Method according to claim 8 or 9, wherein said sample is a tissue sample comprising cells of a subject provided outside the human or animal body or a sample comprising cells cultured *in vitro,* wherein said cells are lysed in order to provide a solution comprising proteins as mentioned in step a).

11. Method according to claim 1 or 2, wherein the method of claims 8 to 10 is used in order to determine the amount of the LSD1 protein in a sample of breast tissue and thus the expression level of LSD1 in said sample.

12. Use of a pharmaceutical composition comprising an LSD1-inhibitor for the treatment of a cancer selected from the group of cancers consisting of breast cancer, lung carcinoma and sarcoma.

13. Use according to claim 12, wherein said pharmaceutical composition comprises said LSD1-inhibitor as the only pharmaceutically active agent.

14. Use according to claim 12, wherein said pharmaceutical composition comprises at least one additional pharmaceutically active agent for the treatment of either breast cancer, lung carcinoma or sarcoma, wherein said at least one additional pharmaceutically active agent is selected from the group comprising Tamoxifen, Fulvestrant (Faslodex®), Trastuzumab (Herceptin®), Lapatinib (Tykerb), Bevacizumab (Avastin), formestane, fadrozole, anastrozole (Arimidex), letrozole (Femara), exemestane (Aromasin), Clodronate, pamidronate, zoledronic acid, ibandronatefor and mixtures thereof the treatment of breast cancer, from the group comprising erlotinib (Tarceva), bevacizumab (Avastin), topotecan (HYCAMTIN®), paclitaxel (Taxol®), docetaxel (Taxotere®), gemcitabine (Gemzar®), and irinotecan (Camptosar®), cyclophosphamide, doxorubicin (Adriamycin®), vincristine (Oncovin®), paclitaxel, cisplatin (Platinol®) and combinations thereof for the treatment of lung carcinoma and from the group comprising Imatinib (Glivec), Trabectedin (Yondelis) and combinations thereof for the treatment of sarcoma.

15. Use according to claims 12 to 14, wherein said inhibitor is selected from the group of MAO-inhibitors comprising trancylpromine, clorgyline, pargyline, iproclozide, isocarboxazid, mebanazine, nialamide, safrazine, befloxatone, cimoxatone and mixtures thereof.
